(19) Europäisches Patentamt
European Patent Office
Office européen des brevets

(11) Publication number: **0 173 276**
**A2**

(12) # EUROPEAN PATENT APPLICATION

(21) Application number: **85110679.9**

(22) Date of filing: **26.08.85**

(51) Int. Cl.[4]: **C 12 Q 1/00**, C 12 Q 1/32, C 12 Q 1/58, C 12 Q 1/34

(30) Priority: **27.08.84 JP 178049/84**

(43) Date of publication of application: **05.03.86 Bulletin 86/10**

(84) Designated Contracting States: **DE FR GB IT**

(71) Applicant: **KYOWA HAKKO KOGYO CO., LTD., 6-1, Ohte-Machi Itchome, Chiyoda-ku Tokyo (JP)**
Applicant: **Kyowa Medex Co. Ltd., 6-1 Ohtemachi Itchome Chiyoda-ku, Tokyo (JP)**

(72) Inventor: **Uwajima, Takayuki, 4-5-10, Naka-machi, Machida-shi Tokyo (JP)**
Inventor: **Fujishiro, Kinya, 3-6-6, Asahi-machi, Machida-shi Tokyo (JP)**
Inventor: **Tsuda, Mitsuru, 20-49, Minamifutsuka-machi, Mishima-shi Shizuoka-ken (JP)**
Inventor: **Tatano, Toshio, 2297-6, Ooka, Numazu-shi Shizuoka-ken (JP)**

(74) Representative: **Casalonga, Axel et al, BUREAU D.A. CASALONGA OFFICE JOSSE & PETIT Baaderstrasse 12-14, D-8000 München 5 (DE)**

(54) **Method for the determination of substrate or enzyme activity.**

(57) The determination of a substrate or an activity of an enzyme in a sample containing ammonia by using a reaction system which forms ammonia can be performed by decomposing ammonia in the sample with an ammonia-decomposing reagent system, adding a reagent system necessary for the formation of ammonia from the substrate or the substrate for the enzyme to form ammonia and determining the formed ammonia.

# TITLE OF THE INVENTION

# METHOD FOR THE DETERMINATION OF SUBSTRATE OR ENZYME ACTIVITY

## Background of the Invention

The present invention relates to a method for the determination of a substrate or an activity of an enzyme in a sample containing ammonia using reaction system which forms ammonia. The feature of the invention is that ammonia in the sample is decomposed prior to the intended assay.

Heretofore, in order to determine the substrate or activity of enzyme in a sample containing ammonia by using a reaction system which forms ammonia, it was necessary to determine ammonia in the sample prior to the intended assay. This method is complicated and it has been demanded to develop a simple method.

As a result of studies of decomposition of ammonia in a sample, it has been found that the intended assay can be achieved by decomposing ammonia in the sample with an enzyme capable of consuming ammonia.

## Summary of the Invention

In accordance with the present invention, the determination of a substrate or an activity of an enzyme in a sample containing ammonia by using a reaction system which forms ammonia can be performed by decomposing ammonia in the sample with an ammonia-decomposing reagent system (hereinafter referred to as "1st step reaction"), adding a reagent system necessary for the formation of ammonia from the substrate or the enzyme to be determined to form ammonia (hereinafter referred to as "2nd step reaction") and determining the formed ammonia.

## Description of the Invention

Any ammonia-decomposing reagent system may be used so long as it has an ability to decompose ammonia. An example of preferred reagent system is a reagent system containing an enzyme capable of consuming ammonia.

As the enzyme, any enzyme may be used so long as it consumes ammonia for the enzyme reaction without NAD (P).

Examples of the enzyme and enzyme reactions thereof are shown below.

GMP synthetase

$$XMP + NH_4^+ + ATP \longrightarrow GMP + AMP + PPi$$

Glutamine synthetase

$$L\text{-glutamate} + NH_4^+ + ATP \longrightarrow L\text{-glutamine} + ADP + Pi$$

Asparagine synthetase

$$L\text{-aspartate} + NH_4^+ + ATP \longrightarrow L\text{-asparagine} + ADP + PPi$$

Tryptophanase

$$Indol + pyruvate + NH_4^+ \longrightarrow L\text{-tryptophan}$$

D(L)-Serine dehydratase

$$Pyruvate + NH_4^+ \longrightarrow D(L)\text{-serine}$$

Homocysteine desulfhydrase

$$2\text{-Oxobutyrate} + H_2S + NH_4^+ \longrightarrow Homocysteine + H_2O$$

Cystathionine γ-lyase

$$2\text{-Oxobutyrate} + L\text{-cysteine} + NH_4^+ \longrightarrow L\text{-cystathionine} + H_2O$$

Ornithine cyclodeaminase

$$L\text{-proline} + NH_4^+ \longrightarrow L\text{-ornithine}$$

Aspartate ammonia-lyase

$$Fumarate + NH_4^+ \longrightarrow L\text{-aspartate}$$

Histidine ammonia-lyase

Urocanate + $NH_4^+$ ⟶ L-histidine

Phenylalanine ammonia-lyase

Trans-cinnamate + $NH_4^+$ ⟶ L-phenylalanine

β-Alanyl-CoA ammonia-lyase

Acrylyl-CoA + $NH_4^+$ ⟶ β-alanyl-CoA

Ethanolamine ammonia-lyase

Acetaldehyde + $NH_4^+$ ⟶ ethanolamine

Methylaspartate ammonia-lyase

Mesaconate + $NH_4^+$ ⟶ L-threo-3-methylaspartate

Tyrosine phenol-lyase

Phenol + pyruvate + $NH_4^+$ ⟶ L-tyrosine + $H_2O$

In carrying out the decomposition of ammonia, ammonia-decomposing enzyme, substrate, etc. necessary for the enzyme reaction are added to the sample.

When a reagent system containing an enzyme is used for decomposing ammonia, the enzyme can be inactivated by suitable means such as heating after completion of the 1st step reaction. The action of the enzyme can also be prevented by applying the following methods.

1. Adjustment of the pH in the 1st and 2nd step reactions

Synthetases, specifically GMP synthetase, are active under alkaline conditions at a pH of 8.0 - 9.5. However, they are inactive at a pH around neutral in a low concentration of substrate. Thus, the decomposition reaction of ammonia is carried out at a pH of 8.0 - 9.5 and 2nd step reaction and the determination reaction of formed ammonia are carried out at a pH of 6.5 - 7.5.

2. Utilization of inhibitor

The inhibitor which acts specifically on the ammonia-decomposing enzyme is added to the 2nd step reaction. As the inhibitor to GMP synthetase, glutamine synthetase and asparagine synthetase, decoinine, methionine sulfoxide, molybdic acid, etc. may be used.

3. Addition of adenosine triphosphatase (ATPase) to the 2nd step reaction

When the ammonia-decomposing enzyme which requires ATP for the enzyme reaction is used, the enzyme reaction is prevented from occurring by decomposing ATP remaining in the reaction solution after completion of the 1st step reaction. ATPase is used in an excess amount for the decomposition of ATP.

After decomposing ammonia in a sample, the enzyme or substrate necessary for the intended assay is added to the reaction mixture to form ammonia and the intended assay can be performed by determining the formed ammonia by a method known per se.

The method of the present invention can be applied to the determination of a substrate which can be introduced to the reaction system which forms ammonia from the substrate by enzyme reaction.

Examples of the substrate are creatinine, L-asparagine, L-glutamine, monocarboxylate, N-carbamyl-β-alanine, L-ureidosuccinic acid, N-formylaspartate, xanthine, cytosine, adenine, guanine, adenosine, cytidine, ADP, 4-aminoimidazol, deoxy-CMP, nitrile and urea.

Further, the method of the present invention can be applied to the determination of an enzyme which can be introduced to the reaction system which forms ammonia from the substrate for the enzyme by enzyme reaction.

Examples of enzyme include creatinine deiminase, asparaginase, glutaminase, amidase, β-ureidopropionase, ureidosuccinase, formiminoaspartate deiminase, guanine deaminase, adenosine deaminase, cytidine deaminase, ADP

deaminase, aminoimidazolase, deoxy CMP deaminase, nitrilase and urease.

The determination of ammonia can be carried out by any system known per se, for example, by reacting ammonia with α-ketoglutamate (hereinafter referred to as α-KG) in the presence of glutamate dehydrogenase (hereinafter referred to as GLDH) and NAD (P) H and determining the decrease of NAD(P)H in the reaction solution photometrically at 340 nm.

The enzyme reaction are schematically shown below.

$$NH_3 + \alpha\text{-KG} + NAD(P)H \xrightarrow{GLDH} \text{glutamic acid} + NAD(P)$$

The determination of ammonia is carried out by determining the total amount of ammonia formed in the 2nd step reaction or by determining the formation rate of ammonia in the 2nd step reaction.

Certain specific embodiments of the invention are illustrated by the following representative examples.

Example 1

Decomposition of ammonia in a sample with GMP synthetase and determination of the activity of guanase in the sample:

Reagent solution A

| | |
|---|---|
| XMP | 10 mM |
| ATP | 10 mM |
| GMP synthetase | 10 U/1.98 ml |
| Tris-HCl buffer (pH 8.5) | 0.1 M |

Reagent solution B

| | |
|---|---|
| Guanine hydrochloride | 10 mM |
| GLDH | 10 U/ml |
| α-KG | 10 mM |
| NADPH | 0.5 mM |
| Tris-HCl buffer (pH 7.0) | 0.1 M |

To 1.98 ml of Reagent solution A were added 20 µl of 0.5 mM ammonia and guanase in the concentration indicated in Table below and the mixture was incubated at 30°C for 5 minutes. To the reaction mixture was added 1 ml of Reagent solution B and the mixture was incubated at 30°C for 5 minutes. The decrease of the absorbancy of the reaction solution at 340 nm was measured to obtain the results shown below.

| Sample No. | Guanase content (U/ml) | ΔE |
|---|---|---|
| 1 | 0.14 | 0.029 |
| 2 | 0.56 | 0.116 |
| 3 | 1.2 | 0.248 |

As a result of calculation according to the following equation, ammonia in the sample was completely decomposed and the activity of guanase in the sample was accurately determined.

$$\text{Guanase activity (U/ml)} = \frac{\Delta E}{6.2} \times \frac{1}{5} \times \frac{3.0}{0.02}$$

ΔE : Decrease of absorbancy by the decrease of NADPH
6.2 : Absorbancy of 1 mM NADPH solution
5 : Reaction time (minute)
3.0 : Amount of reaction solution (ml)
0.02 : Amount of sample (ml)

Separately, 1 ml of 0.5 mM aqueous ammonia was added to 1.98 ml of Reagent solution A and the mixture was incubated at 30°C for 5 minutes. The reaction solution was subjected to ammonia assay using GLDH and no ammonia was detected in the reaction solution.

Example 2

The same procedures as described in Example 1 were repeated except that the following Reagent solution B was used to obtain the results shown below.

Reagent solution B

| | |
|---|---|
| Guanine hydrochloride | 10 mM |
| GLDH | 10 U/ml |
| α-KG | 10 mM |
| NADPH | 0.05 mM |
| ATPase | 10 U/ml |
| Tris HCl buffer (pH 8.0) | 0.1 M |

| Sample No. | ΔE |
|---|---|
| 1 | 0.027 |
| 2 | 0.115 |
| 3 | 0.246 |

Example 3

Decomposition of ammonia in a sample with GMP synthetase and determination of creatinine in the sample using creatinine deiminase:

Reagent solution A

| | |
|---|---|
| XMP | 10 mM |
| ATP | 10 mM |
| GMP synthetase | 10 U/1.98 ml |
| Tris hydrochloride buffer (pH 8.5) | 0.1 M |

Reagent solution B

| | |
|---|---|
| Creatinine deiminase | 10 U/ml |
| GLDH | 10 U/ml |
| α-KG | 10 mM |
| NADPH | 0.5 mM |
| Tris hydrochloride buffer (pH 7.0) | 0.1 M |

To 1.98 ml of Reatent solution A was added 20 µl of a sample containing 0.5 mM ammonia and creatinine in the concentration indicated in Table below and the mixture was incubated at 30°C for 5 minutes. The reaction mixture was added to 1 ml of Reagent solution B and the mixture was incubated at 30°C for 5 minutes. Absorbancy of the reaction solution at 340 nm was measured to obtain the results shown in Table below.

| Sample No. | Creatinine content (mg/ml) | ΔE |
|---|---|---|
| 1 | 0.1 | 0.036 |
| 2 | 0.5 | 0.183 |
| 3 | 1.0 | 0.366 |

As a result of calculation according to the following equation, it was confirmed that ammonia contained in the sample was completely decomposed and creatinine was determined accurately.

$$M = \frac{\Delta E}{6.2} \times \frac{3.0}{0.02} \times \frac{113}{1000}$$

M : Creatinine content (mg/ml)
ΔE : Absorbancy of 1 mM NADPH solution
3.0 : Total amount of reaction solution (ml)
0.02 : Amount of sample (ml)
113 : Molecular weight of creatinine

Separately, 20 µl of a sample containing 0.5 mM ammonia was added to 1.98 ml of Reagent solution A and the mixture was incubated at 30°C for 5 minutes. The reaction solution was subjected to ammonia assay using GLDH and no ammonia was detected in the reaction solution.

Example 4

The same procedures as described in Example 3 were repeated except that the samples containing creatinine in the concentrations indicated in Table below and Reagent solution B shown below were used to obtain the results shown in Table below.

| Sample No. | Creatinine content (mg/ml) | ΔE |
|---|---|---|
| 1 | 0.1 | 0.036 |
| 2 | 0.2 | 0.073 |
| 3 | 0.4 | 0.146 |
| 4 | 0.6 | 0.220 |

Example 5

The same procedures as described in Example 3 were repeated except the samples containing creatinine in the concentrations indicated in Table below and Reagent solution B shown below were used to obtain the results shown in Table below.

Reagent solution B

| | |
|---|---|
| Creatinine deiminase | 10 U/ml |
| GLDH | 10 U/ml |
| α-KG | 10 mM |
| NADPH | 0.05 mM |
| ATPase | 10 U/ml |
| Tris hydrochloride buffer (pH 8.0) | 0.1 M |

| Sample No. | Creatinine content (mg/ml) | ΔE |
|---|---|---|
| 1 | 0.2 | 0.073 |
| 2 | 0.4 | 0.146 |
| 3 | 0.8 | 0.292 |

Example 6

Decomposition of ammonia in a sample with glutamine synthetase and the determination of creatinine in the sample using creatinine deiminase:

Reagent solution A

| | |
|---|---|
| L-glutamic acid | 10 mM |
| ATP | 10 mM |
| glutamine synthetase | 10 U/1.98 ml |
| Tris hydrochloride buffer (pH 8.5) | 0.1 M |

Reagent solution B

| | |
|---|---|
| Creatinine deiminase | 10 U/ml |
| GLDH | 10 U/ml |
| α-KG | 10 mM |
| NADPH | 0.05 mM |
| Methionine sulfoxide | 1 mM |
| Tris hydrochloride buffer (pH 8.0) | 0.1 M |

To 1.98 ml of Reagent solution A was added 20 µl of a sample containing 0.5 mM ammonia and creatinine in the concentration indicated in Table below and the mixture was incubated at 30°C for 5 minutes. To the reaction mixture was added 1 ml of Reagent solution B and the mixture was incubated at 30°C for 5 minutes. The decrease of the absorbancy of the reaction solution at 340 nm was measured to obtain the results shown below.

| Sample No. | Creatinine content (mg/ml) | ΔE |
|---|---|---|
| 1 | 0.2 | 0.072 |
| 2 | 0.4 | 0.144 |
| 3 | 0.8 | 0.290 |

The same calculation method as described in Example 3 was applied, whereby it was confirmed that ammonia in the

sample was completely decomposed and creatinine in the sample was determined accurately.

Example 7

Decomposition of ammonia in a sample with asparagine synthetase and determination of creatinine in the sample using creatinine deiminase:

Reagent solution A

| | |
|---|---|
| L-aspartic acid | 10 mM |
| ATP | 10 mM |
| Asparagine synthetase | 10 U/1.98 ml |
| Tris hydrochloride buffer (pH 8.5) | 0.1 M |

Reagent solution B

| | |
|---|---|
| Creatinine deiminase | 10 U/ml |
| GLDH | 10 U/ml |
| α-KG | 10 mM |
| NADPH | 0.05 mM |
| Molybdic acid | 0.1 mM |
| Tris hydrochloride buffer (pH 8.0) | 0.1 M |

To 1.98 ml of Reagent solution A was added 20 µl of a sample containing 0.5 mM ammonia and creatinine in the concentration indicated in Table below and the mixture was incubated at 30°C for 5 minutes. To the reaction mixture was added 1 ml of Reagent solution B and the mixture was incubated at 30°C for 5 minutes. The decrease of the absorbancy of the reaction solution at 340 nm was measured to obtain the results shown below.

| Sample No. | Creatinine content (mg/ml) | ΔE |
|---|---|---|
| 1 | 0.2 | 0.070 |
| 2 | 0.4 | 0.143 |
| 3 | 0.8 | 0.289 |

The same calculation method as described in Example 3 was applied, whereby it was confirmed that ammonia in the sample was completely decomposed and creatinine in the sample was determined accurately.

What is Claimed is:

1. A method for the determination of a substrate or an activity of an enzyme in a sample containing ammonia by using a reaction system which forms ammonia, which comprises decomposing ammonia in the sample with an ammonia-decomposing reagent system, adding a reagent system necessary for the formation of ammonia from the substrate or the substrate for the enzyme to form ammonia and determining the formed ammonia.

2. A method according to claim 1, wherein said ammonia-decomposing reagent system contains an enzyme capable of consuming ammonia.

3. A method according to claim 1, wherein said determination of ammonia is carried out by reacting ammonia with α-KG in the presence of glutamate dehydrogenase and NAD(P)H and determining the decrease of NAD(P)H.

4. A method according to claim 3, wherein said decrease of NAD(P)H is determined by measuring the absorbancy of the reaction solution at 340 nm.

5. A method according to claim 2, wherein said enzyme is GMP synthetase, glutamine synthetase, asparagine synthetase, tryptophanase, D(L)-serine dehydratase, Homocysteine desulfhydrase, cystathionine γ-lyase, ornithine cyclodeaminase, aspartate ammonia-lyase, histidine ammonia-lyase, phenylalanine ammonia-lyase, β-alanyl-CoA ammonia-lyase, ethanolamine ammonia-lyase, methylaspartate ammonia-lyase or tyrosine phenol-lyase.

6. A method according to claim 1, wherein said substrate to be determined is creatinine, L-asparagine, L-glutamine, monocarboxylate, N-carbamyl-β-alanine, L-ureidosuccinic acid, N-formylaspartate, xanthine, cytosine, adenine, guanine, adenosine, cytidine, ADP, 4-aminoimidazol, deoxy-CMP, nitrile or urea.

7. A method according to claim 1, wherein said enzyme, the activity of which is to be determined is creatinine deiminase, asparaginase, glutaminase, amidase, β-ureidopropionase, ureidosuccinase, formiminoaspartate deiminase, guanine deaminase, adenosine deaminase, cytidine deaminase, ADP deaminase, aminoimidazolase, deoxy CMP deaminase, nitrilase or urease.